# FASCICULE DE BREVET EUROPEEN

(11) **EP 1 722 840 B1**
(45) Date de publication et mention de la délivrance du brevet: **05.10.2011**
(21) Numéro de dépôt: 05736985.2
(22) Date de dépôt: 07.03.2005
(51) Int. Cl.: A61M 5/14, A61J 1/00

(54) **DISPOSITIF DE PERFUSION AUTOMATIQUE POUR L'ADMINISTRATION SEQUENTIELLE DE DEUX SOLUTIONS**
AUTOMATISCHE TRANSFUSIONSVORRICHTUNG FÜR DIE KONSEKUTIVE ABGABE VON ZWEI LÖSUNGEN
AUTOMATIC TRANSFUSION DEVICE FOR CONSECUTIVELY DELIVERING TWO SOLUTIONS

(30) Priorité: 08.03.2004 FR 0450458
(43) Date de publication de la demande: 22.11.2006
(73) Titulaire: MACO PHARMA, 59420 Mouvaux (FR)
(72) Inventeur: BRINON, Thierry, F-95300 Pontoise (FR)
(74) Mandataire: Novagraaf Technologies
(86) Numéro de dépôt international: PCT/FR2005/000538
(87) Numéro de publication internationale: WO 2005/087294

(56) Documents cités:
- GB-A- 1 375 281
- US-A- 4 548 606
- US-A- 5 431 496
- US-A1- 2003 040 708
- US-B1- 6 428 518

## Description

L'invention concerne un dispositif de perfusion par gravité formant un système unitaire en circuit fermé.

Elle s'applique typiquement à la perfusion par gravité destinée à permettre l'administration parentérale de solutions injectables à un patient. Dans un exemple de réalisation particulier, la solution à injecter contient un principe actif nécessitant un dosage précis, tel qu'un agent antimitotique ou antibiotique.

On connaît du document EP-1 060 754 un dispositif de ce type comprenant une poche et des moyens de perfusion en communication fluidique avec un orifice de sortie de ladite poche. La poche comprend deux compartiments indépendants et séparés l'un de l'autre par un système sécable. Le compartiment inférieur contient un soluté de rinçage et le compartiment supérieur, sur lequel est monté l'orifice de sortie, contient une solution médicamenteuse. Le système sécable permet, lors de sa rupture, de mettre en communication fluidique lé compartiment inférieur avec le compartiment supérieur et ainsi de permettre le passage du soluté dans les moyens de perfusion via le compartiment supérieur de sorte à assurer son rinçage.

Un autre document de l'état de la technique antérieur est le GB 1 375 281.

Cette étape de rinçage est une étape nécessaire pour assurer l'injection de la totalité de la solution médicamenteuse. Toutefois, cette étape supplémentaire nécessite la présence du personnel soignant lors de la perfusion puisqu'elle doit être réalisée après la perfusion de la solution médicamenteuse. En outre, si le personnel soignant n'arrête pas la perfusion de la solution médicamenteuse avant que la poche la contenant ne soit complètement vide, l'air contenu dans celle-ci peut être introduit dans les moyens de perfusion. Dans ce cas, lors du rinçage, le volume d'air est administré au patient, provoquant ainsi une embolie gazeuse pouvant entraîner la mort du patient.

L'invention propose de perfectionner un tel dispositif de perfusion de sorte à empêcher toute administration d'air entre l'administration de la solution médicamenteuse et la solution de rinçage par l'intermédiaire des moyens de perfusion. Par ailleurs, le perfectionnement proposé permet un rinçage optimal du dispositif de perfusion de sorte à respecter précisément le dosage de solution médicamenteuse à perfuser. En outre, le rinçage est réalisé de façon automatique, c'est-à-dire sans intervention du personnel soignant ce qui simplifie grandement l'utilisation du dispositif tout en limitant le risque d'erreur inhérent à l'intervention humaine.

A cet effet, l'invention propose un dispositif de perfusion formant un système unitaire en circuit fermé, comprenant une poche formée d'une enveloppe souple définissant un volume intérieur, et des moyens de perfusion en communication fluidique avec le volume intérieur par l'intermédiaire d'une tubulure connectée sur un orifice de sortie de la poche, lendit volume intérieur étant pourvu de moyens de compartimentage qui sont agencés pour former un premier et un deuxième compartiments contenant ou destinés à contenir respectivement une première et une deuxième solutions, lesdits compartiments étant en communication fluidique avec l'orifice respectivement selon un premier et selon un deuxième chemins d'écoulement, le deuxième chemin d'écoulement débouchant dans le premier compartiment. Le premier compartiment comprend un volume supérieur qui est prévu au dessus du deuxième compartiment et un volume inférieur qui est prévu, au moins en partie, au niveau et/ou en dessous du deuxième compartiment, et un obturateur réversible d'écoulement est interposé dans le deuxième chemin d'écoulement, ledit obturateur étant apte, en fonction du différentiel de pression entre l'amont et l'aval du deuxième chemin d'écoulement, à empêcher l'écoulement de la deuxième solution lors de la perfusion du contenu du volume supérieur et à permettre l'écoulement de la deuxième solution lors de la perfusion du contenu du volume inférieur.

L'invention sera comprise grâce à la description qui suit en référence aux dessins annexés, illustrant divers modes de réalisation.
Les figures 1 et 2 représentent de façon schématique deux modes de réalisation d'un dispositif de perfusion selon l'invention.
Les figures 3A, 3B et 3C représentent de façon schématique le dispositif de perfusion selon l'invention à trois étapes différentes de la perfusion.
Les figures 4A et 4B représentent de façon schématique et en coupe transversale un obturateur réversible d'écoulement, respectivement dans sa position fermée et ouverte.
La figure 5 représente partiellement et de façon schématique un mode de réalisation des deux orifices adjacents de la poche des figures 1, 3A, 3B et 3C.
Les figures 6 à 9 représentent partiellement et de façon schématique quatre modes de réalisation de la poche des figures 1, 3A, 3B et 3C.

L'invention concerne un dispositif de perfusion par gravité, notamment destiné à permettre l'administration parentérale de solutions injectables à un patient.

Les termes "supérieur", "inférieur", "horizontal", "vertical", "au dessus" et "en dessous" sont définis par rapport à la position normale d'utilisation du dispositif de perfusion, c'est-à-dire la position du dispositif au moment où le patient va être perfusé. Les termes "amont" et "aval" sont définis par rapport au sens d'écoulement des solutions dans le dispositif de perfusion.

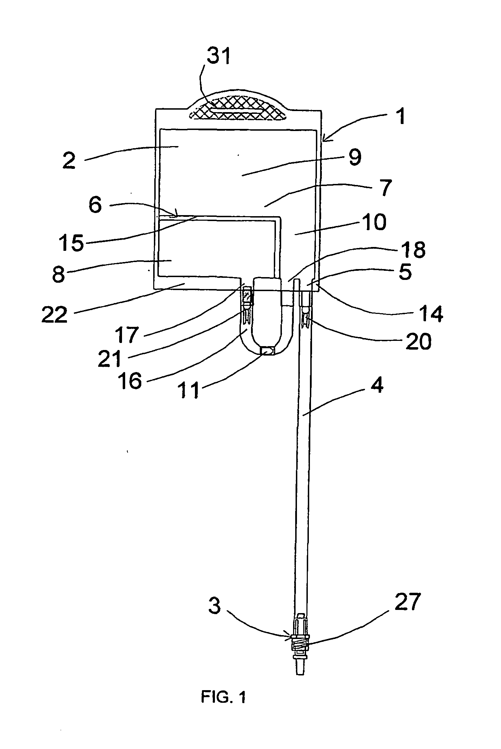

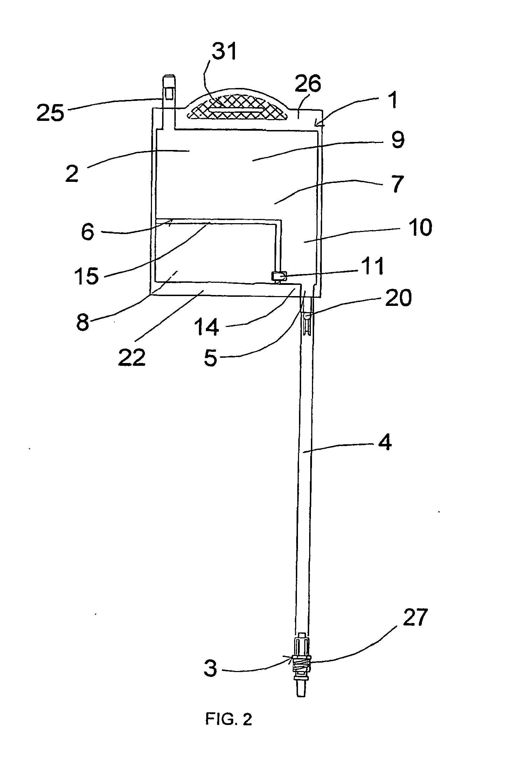

En référence aux figures 1 et 2, le dispositif de perfusion 1 forme un système unitaire en circuit fermé, comprenant une poche 2 formée d'une enveloppe souple définissant un volume intérieur, et des moyens de perfusion 3 en communication fluidique avec le volume intérieur par l'intermédiaire d'une tubulure 4 connectée sur un orifice de sortie 5 de la poche 2.

Dans ce dispositif de perfusion 1, les poches et les tubulures du système sont réalisées notamment en un matériau thermoplastique souple et stérilisable tel que le polychlorure de vinyle ou une polyoléfine.

Le dispositif de perfusion 1 selon l'invention forme un système unitaire en circuit fermé, c'est-à-dire un système prêt à l'emploi, dont les différents éléments le constituant sont pré-connectés. Les éléments étant connectés de fabrication, on évite ainsi une étape de connexion préalablement à l'utilisation du dispositif, qui constitue une manipulation supplémentaire entraînant une perte de temps et un risque de contamination.

Le volume intérieur de enveloppe souple formant la poche 2 est pourvu de moyens de compartimentage 6 qui sont agencés pour former un premier et un deuxième compartiments 7,8 contenant ou destinés à contenir respectivement une première et une deuxième solutions.

Les première et deuxième solutions sont des solutions injectables. Elles sont placées, respectivement, dans les premier et deuxième compartiments 7,8, au moment de la fabrication ou introduits dans le dispositif de perfusion 1 au moment de l'emploi par un orifice d'entrée/sortie (non représenté) de chacun des compartiments.

Par exemple, le premier compartiment 7 contient une solution contenant un médicament, tel qu'un antibiotique ou un antimitotique et le deuxième compartiment 8 contient une solution de rinçage. Des exemples de solution de rinçage comprennent des solutions de chlorure de sodium, glucose et solution de Ringer.

Les compartiments 7,8 sont en communication fluidique avec l'orifice de sortie 5 respectivement selon un premier et selon un deuxième chemins d'écoulement, le deuxième chemin d'écoulement débouchant dans le premier compartiment. Dans le dispositif selon l'invention, le premier compartiment 7 comprend un volume supérieur 9 qui est prévu au dessus du deuxième compartiment 8 et un volume inférieur 10 qui est prévu, au moins en partie, au niveau et/ou en dessous du deuxième compartiment.

Un obturateur réversible 11 d'écoulement est interposé dans le deuxième chemin d'écoulement, ledit obturateur étant apte, en fonction du différentiel de pression entre l'amont et l'aval du deuxième chemin d'écoulement, à empêcher l'écoulement de la deuxième solution lors de la perfusion du contenu du volume supérieur 9 et à permettre l'écoulement de la deuxième solution lors de la perfusion du contenu du volume inférieur 10.

L'obturateur réversible d'écoulement 11 empêche l'écoulement de fluide dans le deuxième chemin d'écoulement ou autorise cet écoulement dans une seule direction, à savoir du deuxième compartiment 8 vers le premier compartiment 7.

Selon une réalisation particulière, l'obturateur réversible 11 est de type clapet de non retour qu'un excès de pression fait ouvrir momentanément ou fermer si la pression s'exerce dans le sens inverse de l'écoulement du fluide.

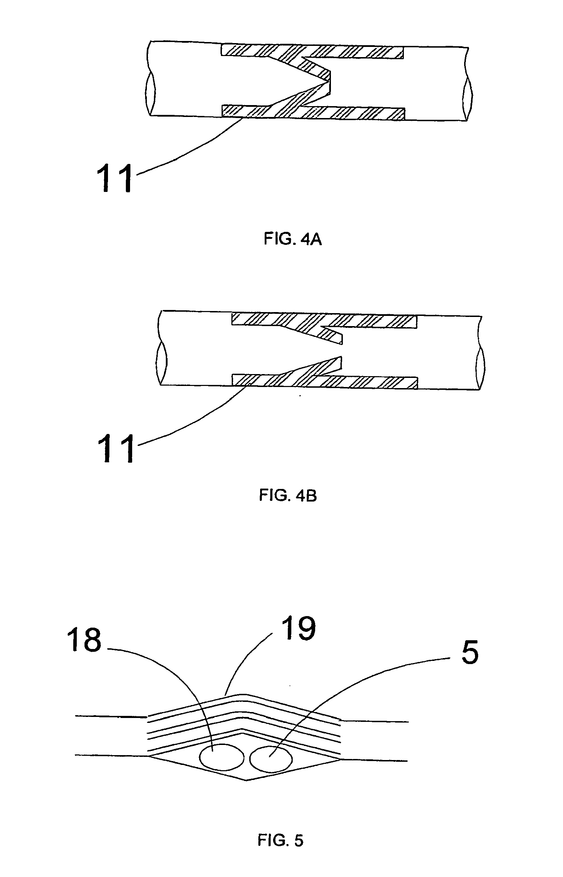

Un exemple particulier d'obturateur réversible, représenté sur les figures 4A et 4B, respectivement en position fermée et ouverte, est un clapet dit "bec de canard".

Le principe de fonctionnement du dispositif de perfusion 1 repose sur le principe des "vases communicants" selon lequel les pressions en amont et aval du chemin d'écoulement et les niveaux des solutions dans les premier et deuxième compartiments 7,8 tendent à s'équilibrer automatiquement.

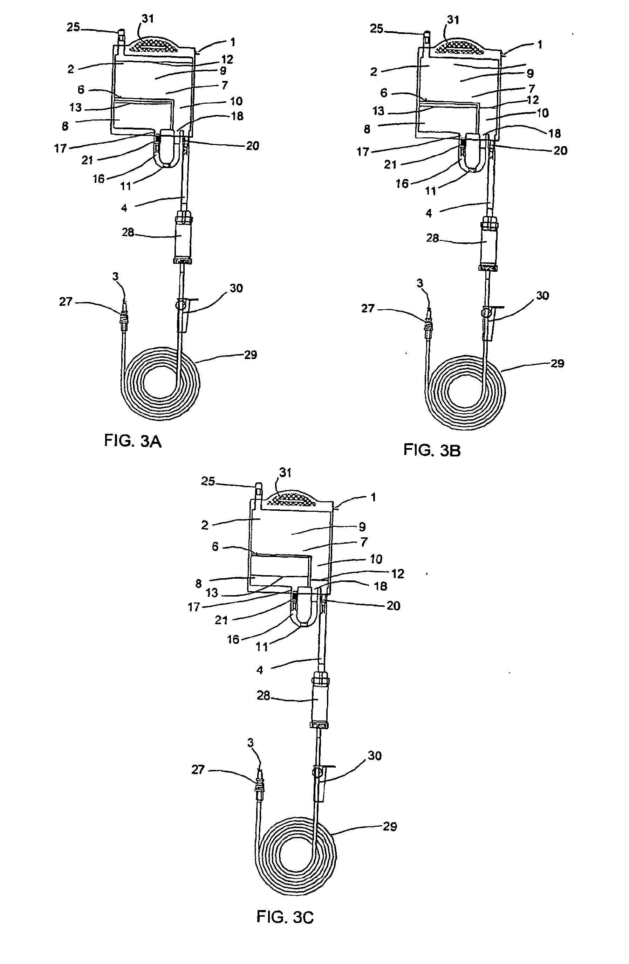

Les différentes étapes de la perfusion sont représentées sur les figures 3A, 3B et 3C.

Dans une première phase représentée sur la figure 3A, correspondant au début de la perfusion, le niveau 12 de la solution contenue dans le volume supérieur 9 du premier compartiment 7 est au dessus du niveau 13 de la deuxième solution contenue dans le deuxième compartiment 8, et la pression en aval du deuxième chemin d'écoulement est supérieure à la pression en amont.

Dans cette première phase, puisque la pression en aval du deuxième chemin d'écoulement est supérieure à celle en amont, l'obturateur réversible 11, placé dans le deuxième chemin d'écoulement, est fermé. Par conséquent, la deuxième solution ne peut pas s'écouler vers le premier compartiment 7 ni la première solution dans le deuxième compartiment 8. Ainsi, seule la première solution s'écoule dans les moyens de perfusion 3 par le premier chemin d'écoulement.

Au cours de la première phase, la première solution s'écoule seule dans les moyens de perfusion 3 jusqu'à ce que le niveau 12 de la solution dans le compartiment 7 atteigne le niveau 13 de la solution contenue dans le compartiment 8 (figure 3B). A ce moment, l'obturateur réversible 11 est à l'équilibre.

Le passage à la deuxième phase représentée sur la figure 3C est ensuite automatique. Aucune action extérieure n'est nécessaire.

Dans cette deuxième phase, la première solution continuant à s'écouler par gravité dans les moyens de perfusion 3, la pression en aval du deuxième chemin d'écoulement est inférieure à la pression en amont puisque le niveau 12 de la première solution est en dessous du niveau 13 de la deuxième solution.

L'obturateur réversible 11 est alors ouvert. La deuxième solution contenue dans le deuxième compartiment 8 s'écoule librement dans le deuxième chemin d'écoulement vers le premier compartiment 7. La solution contenue dans le premier compartiment comprenant la première et deuxième solutions s'écoule par le premier chemin d'écoulement dans les moyens de perfusion 3.

Dans le cas particulier où la première solution est une solution médicamenteuse et la deuxième solution est une solution de rinçage, cette deuxième phase constitue l'étape de rinçage.

En effet, dans cette phase, la solution de rinçage contenue dans le deuxième compartiment 8 s'écoule par le deuxième chemin d'écoulement dans le volume inférieur du premier compartiment contenant la solution médicamenteuse, provoquant la dilution de la solution médicamenteuse. La solution qui passe dans le premier compartiment 7 puis dans les moyens de perfusion 3 est alors une solution médicamenteuse diluée avec la solution de rinçage.

A la fin de la perfusion, la solution médicamenteuse a subit une dilution en série si bien que la quantité de médicament restant en solution est minime.

Ainsi, le dispositif de perfusion 1 selon l'invention permet l'administration automatique et séquentielle d'une solution de médicament et d'une solution de rinçage. Les derniers millilitres de solution passant dans les moyens de perfusion 3 comprenant une quantité négligeable de médicament, on peut considérer que le rinçage de la partie inférieure du premier compartiment 7 et des moyens de perfusion 3 est complet à la fin de la perfusion.

Aucun volume d'air ne peut être administré au patient entre la perfusion de la solution médicamenteuse et la solution de rinçage, puisque l'administration des solutions est réalisée de façon séquentielle et sans interruption de l'écoulement de solution dans les moyens de perfusion 3.

En outre, le passage de la première à la deuxième phase est instantané, ce qui permet d'éviter la stagnation de la solution médicamenteuse dans le dispositif de perfusion 1 et dans l'abord veineux du patient. Ainsi on évité une éventuelle absorption du médicament par les matériaux constituant le dispositif de perfusion 1 et par l'abord veineux du patient, en particulier lors de la perfusion de molécules agressives.

Enfin, puisque les deux solutions sont administrées séquentiellement et automatiquement, le risque d'inversion dans l'ordre d'administration des solutions est éliminé.

Selon une réalisation particulière représentée sur les figures 1 et 2, l'orifice de sortie 5 du dispositif de perfusion 1 est prévu sur le bord inférieur 14 du premier compartiment 7, au niveau le plus bas du volume inférieur 10 de sorte à définir un premier chemin d'écoulement direct.

Ainsi, à la fin de la perfusion, le premier compartiment 7 est totalement vide. Seul un volume minime de deuxième solution est susceptible de rester dans le deuxième chemin d'écoulement. Ainsi, lorsque la première solution contient un médicament, la totalité du médicament est perfusé au patient.

En référence avec les figures 1 et 2, les moyens de compartimentage 6 du volume intérieur de l'enveloppe souple sont formés d'un cordon de soudure 15 comprenant une partie horizontale et une partie verticale, le volume supérieur 9 étant formé au dessus de la partie horizontale et le volume inférieur 10 en regard de la partie verticale.

Le deuxième compartiment 8 est alors de forme rectangulaire et le premier compartiment 7 en forme de L inversé.

Selon une première réalisation représentée sur la figure 1, le deuxième chemin d'écoulement est réalisé au moyen d'une tubulure 16 dont l'extrémité amont est connectée à un orifice de sortie 17 du deuxième compartiment 7 et dont l'extrémité aval est connectée à un orifice d'entrée 18 du premier compartiment 7, l'obturateur réversible 11 étant disposé dans ladite tubulure 16.

Dans cette réalisation, les orifices d'entrée 18 et de sortie 5 du premier compartiment 7 sont adjacents.

Notamment, les orifices comprennent un élément volumique sensiblement rigide. En particulier, les orifices d'entrée 18 et de sortie 5 sont formés d'une seule pièce rigide et moulée 19 telle que représentée sur la figure 5. Cette pièce facilite la fabrication des orifices adjacents. De plus, la pièce 19, ayant une certaine épaisseur, empêche le collabage de l'enveloppe souple.

Dans cette réalisation, les orifices de sortie 5, 17 du premier et du deuxième compartiment 7,8 sont pourvus d'un organe sécable 20,21 de type ouvre-circuit.

Au moment de l'utilisation du dispositif de perfusion 1, il suffit de casser les deux organes sécables 20, 21 pour perfuser la première solution au patient puis automatiquement la deuxième solution.

Selon une autre réalisation représentée sur la figure 2, le deuxième chemin d'écoulement est formé au travers des moyens de compartimentage 6.

Lorsque les moyens de compartimentage 6 sont formés d'un cordon de soudure 15 comprenant une partie verticale et horizontale, l'obturateur 11 est placé au travers de la partie verticale du cordon de soudure 3, notamment au voisinage de l'extrémité inférieure de la partie verticale.

Dans cette autre réalisation, l'orifice de sortie du premier compartiment 7 est pourvu d'un organe sécable 20 de type ouvre-circuit.

Comme représenté sur les figures 1 et 2, le bord inférieur 22 du deuxième compartiment 8 est sensiblement dans le prolongement du bord inférieur 14 du premier compartiment.

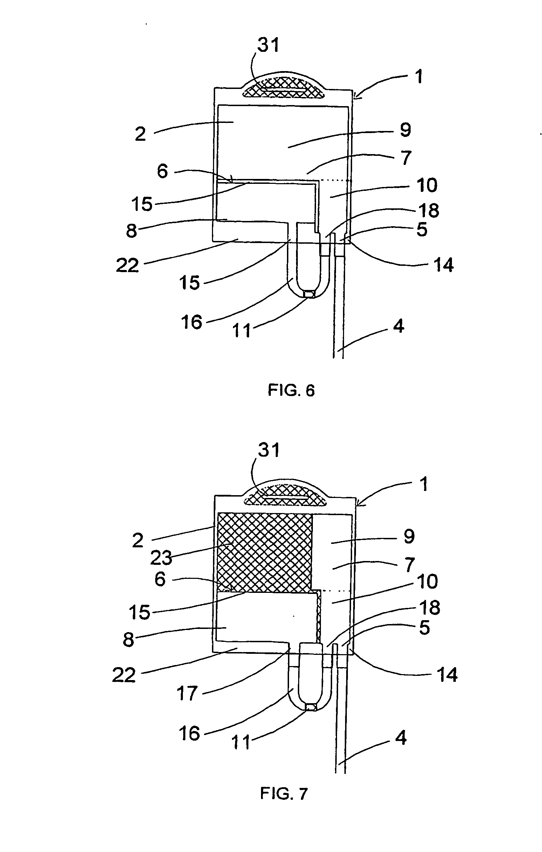

Selon la figure 6, le bord inférieur 22 du deuxième compartiment 8 est décalé vers le haut par rapport au bord inférieur 14 du premier compartiment 7. Dans cette configuration, le deuxième compartiment 8 est vidé en totalité de la deuxième solution à la fin de la perfusion. Le rinçage du volume inférieur 10 du premier compartiment 7 et des moyens de perfusion est alors optimal puisque toute la solution de rinçage est utilisée.

Dans un exemple particulier, le premier compartiment 7 est destiné à contenir un volume de première solution, notamment compris entre 50 et 1000 mL et le deuxième compartiment 8 un volume de deuxième solution, notamment de 100 mL.

Lorsque le volume de la première solution contenue dans le volume supérieure 9 du premier compartiment 7 est inférieur au volume de la deuxième solution dans le deuxième compartiment 8, le niveau de la première solution peut se retrouver juste au dessus du niveau de la deuxième solution.

Selon la figure 7, pour augmenter le différentiel de pression entre l'amont et l'aval du deuxième chemin d'écoulement et assurer le bon fonctionnement de l'obturateur réversible d'écoulement 11, les moyens de compartimentage 6 comprennent en outre une zone soudée 23 qui est prévue dans le volume supérieur.

Cette zone soudée 23 réduit le volume supérieur 9 du premier compartiment 7. Ainsi, au début de la perfusion, le niveau de la première solution est très au-dessus du deuxième compartiment.

En particulier, les moyens de compartimentage 6 définissent, avec le bord vertical de la poche 2 en regard du volume inférieur 10, un volume inférieur de forme rectangulaire de largeur la plus étroite possible de sorte à minimiser le volume inférieur 10 du premier compartiment 7 et ainsi minimiser le volume de solution médicamenteuse à diluer lors de l'étape de rinçage.

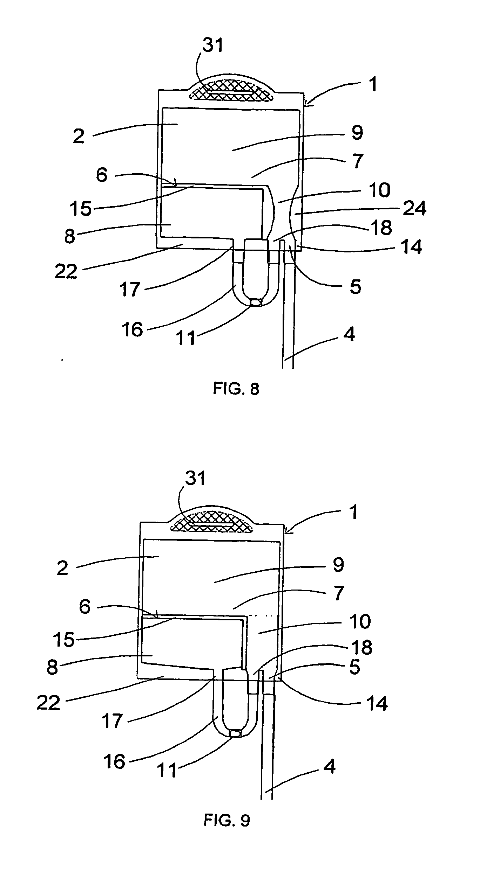

Selon la figure 8, le volume inférieur 10 est délimité latéralement par deux parois 24 incurvées vers l'intérieur. Cette forme particulière réduit le volume inférieur 10 du premier compartiment 7 et permet d'obtenir une largeur de bord inférieur du premier compartiment suffisante pour placer l'orifice 5 et éventuellement l'orifice 18.

En outre, l'incurvation des parois permet d'une part, à la solution contenue dans le volume supérieur 9 de s'écouler facilement dans le volume inférieur 10 et d'autre part, de faciliter la mise en place de l'orifice de sortie 5.

De manière générale, afin d'obtenir une étape de rinçage efficace, le deuxième compartiment 8 présente un volume de l'ordre de 4 à 50 fois supérieur à celui du volume inférieur 10.

Selon la figure 9, les bords inférieurs 14, 22 des premier et/ou deuxième compartiments 7,8 sont inclinés suivant respectivement le premier et le deuxième chemins d'écoulement.

Selon la figure 2, le premier compartiment 7 est pourvu d'un site d'injection 25 qui permet la reconstitution d'une solution médicamenteuse avant la perfusion.

Par exemple, les deux compartiments sont pré-remplis d'une solution injectable de type solution de rinçage. En particulier, les deux compartiments contiennent la même solution. Au moment de l'emploi, un médicament est reconstitué dans la solution contenue dans le premier compartiment 7 à l'aide d'un dispositif de transfert par l'intermédiaire du site d'injection 25 placé sur le premier compartiment 7. Un dispositif de transfert approprié est décrit dans le document EP-A- 1 034 772.

Le site d'injection 25 est notamment prévu sur le bord supérieur 26 du premier compartiment 7. Ainsi, aucun volume mort de solution n'est piégé au niveau du site d'injection 25. Ce positionnement contribue à l'injection de la totalité de la première solution, notamment contenant un médicament.

Comme représentés sur les figures 3A, 3B et 3C, les moyens de perfusion 3 comprennent un moyen de raccordement 27 au circuit veineux du patient, telle qu'une aiguille ou un raccord pour cathéter, notamment un raccord mâle de type cône Luer.

Les moyens de perfusion 3 comprennent en outre une chambre compte-gouttes 28, qui constitue un moyen de contrôle du débit de la perfusion. La chambre compte-gouttes est connectée à une extrémité à la tubulure 4 et à l'autre extrémité au moyen de raccordement 27 par l'intermédiaire d'une tubulure 29.

Cette chambre compte-gouttes est également appropriée pour amorcer la perfusion de la première solution, c'est-à-dire envoyer de la solution dans les moyens de perfusion.

Un régulateur de débit à roulette 30 pouvant contrôler et stopper le débit de la solution à perfuser est placé sur la tubulure de perfusion 29, notamment en aval de la chambre compte-gouttes 28.

La poche 2 comprend un moyen de suspension 31 qui est prévu pour maintenir ladite poche 2 dans une position de perfusion dans laquelle le volume supérieur 9 est disposé respectivement au dessus du volume inférieur 10 et au dessus du deuxième compartiment 8.

Enfin, afin d'augmenter la pression à l'intérieur du deuxième compartiment et faciliter l'ouverture de l'obturateur réversible, le deuxième compartiment contient un volume d'air.

Dans un exemple particulier de mise en oeuvre du dispositif de perfusion 1 tel que représenté sur la figure 3 dans lequel les deux compartiments sont pré-remplis d'une même solution injectable tel que du chlorure de sodium, on réalise les étapes successives suivantes :
- casser l'organe sécable 20 du premier compartiment 7 afin de permettre l'écoulement de la première solution dans les moyens de perfusion,
- réaliser l'amorçage de la première solution dans les moyens de perfusion 3,
- reconstituer la solution médicamenteuse dans le premier compartiment à l'aide du site d'injection 25, et
- casser l'organe sécable 21 du deuxième compartiment afin de permettre le passage de la deuxième solution dans le deuxième chemin d'écoulement.

L'étape d'amorçage permet le rinçage de l'abord veineux du patient avant la perfusion. Notamment, un volume additionnel de première solution est prévu afin de reconstituer la solution médicamenteuse dans un volume standard de solution.

## Revendications

1. Dispositif de perfusion (1) formant un système unitaire en circuit fermé, comprenant une poche (2) formée d'une enveloppe souple définissant un volume intérieur, et des moyens de perfusion (3) en communication fluidique avec le volume intérieur par l'intermédiaire d'une tubulure (4) connectée sur un orifice (5) de sortie de la poche (2), ledit volume intérieur étant pourvu de moyens de compartimentage (6) qui sont agencés pour former un premier et un deuxième compartiments (7,8) contenant ou destinés à contenir respectivement une première et une deuxième solutions, lesdits compartiments (7,8) étant en communication fluidique avec l'orifice (5) respectivement selon un premier et selon un deuxième chemins, d'écoulement, le deuxième chemin d'écoulement débouchant dans le premier compartiment (7), le dit premier comportement (7) comportant un volume supérieur (9) qui est prévu au dessus du deuxième compartiment (8) et un volume inférieur (10) qui est prévu, au moins en partie, au niveau et/ou en dessous du deuxième - compartiment (8), l'obturateur réversible d'écoulement (11) étant interposé dans le deuxième chemin d'écoulement, ledit obturateur étant apte, en fonction du différentiel de pression entre l'amont et l'aval du deuxième chemin d'écoulement, à empêcher l'écoulement de la deuxième solution lors de la perfusion du contenu du volume supérieur (9) et à permettre l'écoulement de la deuxième solution lors de la perfusion du contenu du volume inférieur (10).

2. Dispositif selon la revendication 9, **caractérisé en ce que** l'orifice de sortie (5) est prévu sur le bord inférieur (14) du premier compartiment (7), au niveau le plus bas du volume inférieur (10) de sorte à définir un premier chemin d'écoulement direct

3. Dispositif selon la revendication 1 ou 2, **caractérisé en ce que** les moyens de compartimentage (6) sont formés d'un cordon de soudure (15) comprenant une partie horizontale et une partie verticale, le volume supérieur (9) étant formé au dessus de la partie horizontale et le volume inférieur (10) en regard de la partie verticale.

4. Dispositif selon l'une quelconque des revendications 1 à 3, **caractérisé en ce que** le deuxième chemin d'écoulement est réalisé au moyen d'une tubulure (16) dont l'extrémité amont est connectée à un orifice de sortie (17) du deuxième compartiment (8) et dont l'extrémité aval est connectée à un orifice d'entrée (18) du premier compartiment (7), l'obturateur (11) étant disposé dans ladite tubulure (16).

5. Dispositif selon la revendication 4, **caractérisé en ce que** les orifices d'entrée (18) et de sortie (5) du premier compartiment sont adjacents.

6. Dispositif selon la revendication 5, **caractérisé en ce que** les orifices comprennent un élément volumique (19) sensiblement rigide.

7. Dispositif selon l'une quelconque des revendications 1 à 6, **caractérisé en ce que** le deuxième chemin d'écoulement est formé au travers des moyens de compartimentage (6).

8. Dispositif selon la revendication 7 lorsqu'elle dépend de la revendication 3, **caractérisé en ce que** l'obturateur (11) est placé au travers de la partie verticale du cordon de soudure (15).

9. Dispositif selon la revendication 8, **caractérisé en ce que** l'obturateur (11) est prévu au voisinage de l'extrémité inférieure de la partie verticale.

10. Dispositif selon l'une quelconque des revendications 1 à 9, **caractérisé en ce que** les orifices de sortie (5, 17) sont pourvus d'un organe sécable de type ouvre-circuit (20,21).

11. Dispositif selon l'une quelconque des revendications 1 à 10, **caractérisé en ce que** le bord inférieur (22) du deuxième compartiment (8) est sensiblement dans le prolongement du bord inférieur (14) du premier compartiment (7).

12. Dispositif selon l'une quelconque des revendications 1 à 10, **caractérisé en ce que** le bord inférieur (22) du deuxième compartiment (8) est décalé vers le haut par rapport au bord inférieur (14) du premier compartiment (7).

13. Dispositif selon l'une quelconque des revendications 1 à 12, **caractérisé en ce que** les moyens de compartimentage (6) comprennent en outre une zone soudée (23) qui est prévue dans le volume supérieur (9).

14. Dispositif selon l'une quelconque des revendications 1 à 13, **caractérisé en ce que** le volume inférieur (10) est délimité latéralement par deux parois (24) incurvées vers l'intérieur.

15. Dispositif selon l'une quelconque des revendications 1 à 14, **caractérisé en ce que** le deuxième compartiment (8) présente un volume de l'ordre de 4 à 50 fois supérieur à celui du volume inférieur (10).

16. Dispositif selon l'une quelconque des revendications 1 à 15, **caractérisé en ce que** les bords inférieurs (14, 22) des premier et/ou deuxième compartiments (7,8) sont inclinés suivant respectivement le premier et le deuxième chemins d'écoulement.

17. Dispositif selon l'une quelconque des revendications 1 à 16, **caractérisé en ce que** le premier compartiment (7) est pourvu d'un site d'injection (25).

18. Dispositif selon la revendication 17, **caractérisé en ce que** le site d'injection (25) est prévu sur le bord supérieur (26) du premier compartiment (7).

19. Dispositif selon l'une quelconque des revendications 1 à 18, **caractérisé en ce que** les moyens de perfusion (3) comprennent en outre une chambre compte-gouttes (28).

20. Dispositif selon l'une quelconque des revendications 1 à 19, **caractérisé en ce que** la poche (2) comprend un moyen de suspension (31) qui est prévu pour maintenir ladite poche (2) dans une position de perfusion dans laquelle le volume supérieur (9) est disposé respectivement au dessus du volume inférieur (10) et au dessus du deuxième compartiment (8).

21. Dispositif selon l'une quelconque des revendications 1 à 20, **caractérisé en ce que** le deuxième compartiment (8) contient un volume d'air.

## Claims

1. Perfusion device (1) forming a unitary closed-circuit system including a bag (2) consisting of a flexible casing defining an interior space, and perfusion means (3) in fluid communication with the interior space by means of a tubing (4) connected to an outlet opening (5) of the bag (2), said interior space being provided with compartmentation means (6) which are designed to form first and second compartments (7, 8) containing or intended to contain first and second solutions, respectively, said compartments (7, 8) being in fluid communication with the opening (5) via first and second flow paths, respectively, the second flow path leading into the first compartment (7), said first compartment (7) comprising an upper space (9) which is provided above the second compartment (8) and a lower space (10) which is at least partially provided at the same level as and/or below the second compartment (8), the reversible flow valve (11) being positioned within the second flow path, said valve being capable of, based on the pressure differential between the upstream and downstream portions of the second flow path, preventing the second solution from flowing out during perfusion of the content of the upper space (9) and of enabling the second solution to flow out during perfusion of the content of the lower space (10).

2. Device according to claim 1, **characterised in that** the outlet opening (5) is provided on the lower edge (14) of the first compartment (7), at the lowest level of the interior space (10) so as to define a first direct flow path.

3. Device according to claim 1 or 2, **characterised in that** the compartmentation means (6) consist of a weld seam (15) including a horizontal portion and a vertical portion, the upper space (9) being formed above the horizontal portion and the lower space (10) opposite the vertical portion.

4. Device according to any of claims 1 to 3, **characterised in that** the second flow path is produced by means of a tubing (16) the upstream end of which is connected to an outlet opening (17) of the second compartment (8) and the downstream end of which is connected to an inlet opening (18) of the first compartment (7), the valve (11) being arranged in said tubing (16).

5. Device according to claim 4, **characterised in that** the inlet (18) and outlet (5) openings of the first compartment are adjacent to each other.

6. Device according to claim 5, **characterised in that** the openings include a substantially rigid volume element (19).

7. Device according to any of claims 1 to 6, **characterised in that** the second flow path is formed through the compartmentation means (6).

8. Device according to claim 7 when same depends on claim 3, **characterised in that** the valve (11) is placed through the vertical portion of the weld seam (15).

9. Device according to claim 8, **characterised in that** the valve (11) is provided in the vicinity of the lower end of the vertical portion.

10. Device according to any of claims 1 to 9, **characterised in that** the outlet openings (5, 17) are provided with a breakable member of the open-circuit type (20, 21).

11. Device according to any of claims 1 to 10, **characterised in that** the lower edge (22) of the second compartment (8) is substantially in line with the lower edge (14) of the first compartment (7).

12. Device according to any of claims 1 to 10, **characterised in that** the lower edge (22) of the second compartment (8) is offset upwardly relative to the lower edge (14) of the first compartment (7).

13. Device according to any of claims 1 to 12, **characterised in that** the compartmentation means (6) further include a welded area (23) which is provided in the upper space (9).

14. Device according to any of claims 1 to 13, **characterised in that** the lower space (10) is defined laterally by two inwardly curved walls (24).

15. Device according to any of claims 1 to 14, **characterised in that** the second compartment (8) has a volume of the order of 4 to 50 times greater than that of the lower space (10).

16. Device according to any of claims 1 to 15, **characterised in that** the lower edges (14, 22) of the first and/or second compartments (7, 8) are inclined along the first and second flow paths, respectively.

17. Device as claimed in any of claims 1 to 16, **characterised in that** the first compartment (7) is provided with an injection site (25).

18. Device according to claim 17, **characterised in that** the injection site (25) is provided on the upper edge (26) of the first compartment (7).

19. Device according to any of claims 1 to 18, **characterised in that** the perfusion means (3) further include a drip chamber (28).

20. Device according to any of claims 1 to 19, **characterised in that** the bag (2) includes a suspension means (31) which is provided for holding said bag (2) in a perfusion position in which the upper space (9) is arranged above the lower space (10) and above the second compartment (8), respectively.

21. Device according to any of claims 1 to 20, **characterised in that** the second compartment (8) contains an air space.

## Patentansprüche

1. Infusionsvorrichtung (1), die ein einheitliches System mit geschlossenem Kreislauf bildet, das einen Beutel (2), der von einer weichen Hülle gebildet wird, die ein Innenvolumen definiert, und Infusionsmittel (3) in Flüssigkeitsverbindung mit dem Innenvolumen mittels einer Leitung (4), die an eine Austrittsöffnung (5) des Beutels (2) angeschlossen ist, umfasst, wobei das besagte Innenvolumen mit Abteilmitteln (6) versehen ist, die dazu eingerichtet sind, eine erste und eine zweite Kammer (7, 8) zu bilden, die eine erste bzw. eine zweite Lösung enthalten oder dazu bestimmt sind, eine erste bzw. eine zweite Lösung zu enthalten, wobei die besagten Kammern (7, 8) gemäß einem ersten bzw. einem zweiten Strömungsweg mit der Öffnung (5) in Flüssigkeitsverbindung stehen, wobei der zweite Strömungsweg in der ersten Kammer (7) mündet, wobei die besagte erste Kammer (7) ein oberes Volumen (9), das über der zweiten Kammer (8) vorgesehen ist, und ein unteres Volumen (10), das mindestes zum Teil auf Höhe und/oder unter der zweiten Kammer (8) vorgesehen ist, umfasst, wobei das Rückschlagventil (11) in den zweiten Strömungsweg eingesetzt ist, wobei das besagte Ventil dazu geeignet ist, in Abhängigkeit vom Druckunterschied zwischen dem stromaufwärtigen Teil und dem stromabwärtigen Teil des zweiten Strömungswegs das Strömen der zweiten Lösung bei der Infusion des Inhalts des oberen Volumens (9) zu verhindern und das Strömen der zweiten Lösung bei der Infusion des Inhalts des unteren Volumens (10) zuzulassen.

2. Vorrichtung nach Anspruch 1, **dadurch gekennzeichnet, dass** die Austrittsöffnung (5) am unteren Rand (14) der ersten Kammer (7) auf Höhe des tiefsten Punkts des unteren Volumens (10) vorgesehen ist, um einen ersten direkten Strömungsweg zu definieren.

3. Vorrichtung nach Anspruch 1 oder 2, **dadurch gekennzeichnet, dass** die Abteilmittel (6) von einer Schweißnaht (15) gebildet werden, die einen horizontalen Teil und einen vertikalen Teil umfasst, wobei das obere Volumen (9) über dem horizontalen Teil und das untere Volumen (10) gegenüber dem vertikalen Teil ausgebildet sind.

4. Vorrichtung nach einem der Ansprüche 1 bis 3, **dadurch gekennzeichnet, dass** der zweite Strömungsweg mittels einer Leitung (16) umgesetzt wird, dessen stromaufwärtiges Ende mit einer Austrittsöffnung (17) der zweiten Kammer (8) verbunden ist und dessen stromabwärtiges Ende mit einer Eintrittsöffnung (18) der ersten Kammer (7) verbunden ist, wobei das Ventil (11) in der besagten Leitung (16) angeordnet ist.

5. Vorrichtung nach Anspruch 4, **dadurch gekennzeichnet, dass** die Eintrittsöffnung (18) und die Austrittsöffnung (5) der ersten Kammer aneinandergrenzen.

6. Vorrichtung nach Anspruch 5, **dadurch gekennzeichnet, dass** die Öffnungen ein im Wesentlichen steifes Volumenelement (19) umfassen.

7. Vorrichtung nach einem der Ansprüche 1 bis 6, **dadurch gekennzeichnet, dass** der zweite Strömungsweg durch Abteilmittel (6) gebildet wird.

8. Vorrichtung nach Anspruch 7, wenn dieser von Anspruch 3 abhängt, **dadurch gekennzeichnet, dass** das Ventil (11) durch den vertikalen Teil der Schweißnaht (15) platziert wird.

9. Vorrichtung nach Anspruch 8, **dadurch gekennzeichnet, dass** das Ventil (11) in der Nähe des unteren Endes des vertikalen Teils vorgesehen ist.

10. Vorrichtung nach einem der Ansprüche 1 bis 9, **dadurch gekennzeichnet, dass** die Austrittsöffnungen (5, 17) mit einem teilbaren Organ des Kreislauföffnungstyps (20, 21) versehen sind.

11. Vorrichtung nach einem der Ansprüche 1 bis 10, **dadurch gekennzeichnet, dass** der untere Rand (22) der zweiten Kammer (8) im Wesentlichen im Fortsatz des unteren Rands (14) der ersten Kammer (7) ist.

12. Vorrichtung nach einem der Ansprüche 1 bis 10, **dadurch gekennzeichnet, dass** der untere Rand (22) der zweiten Kammer (8) in Bezug auf den unteren Rand (14) der ersten Kammer (7) nach oben versetzt ist.

13. Vorrichtung nach einem der Ansprüche 1 bis 12, **dadurch gekennzeichnet, dass** die Abteilmittel (6) außerdem eine Schweißzone (23) umfassen, die in dem oberen Volumen (9) vorgesehen ist.

14. Vorrichtung nach einem der Ansprüche 1 bis 13, **dadurch gekennzeichnet, dass** das untere Volumen (10) seitlich durch zwei nach innen gewölbte Wände (24) begrenzt wird.

15. Vorrichtung nach einem der Ansprüche 1 bis 14, **dadurch gekennzeichnet, dass** die zweite Kammer (8) ein Volumen in der Größenordnung von dem 4- bis 50-fachen des unteren Volumens (10) aufweist.

16. Vorrichtung nach einem der Ansprüche 1 bis 15, **dadurch gekennzeichnet, dass** die unteren Ränder (14, 22) der ersten und/oder der zweiten Kammer (7, 8) gemäß dem ersten bzw. dem zweiten Strömungsweg geneigt sind.

17. Vorrichtung nach einem der Ansprüche 1 bis 16, **dadurch gekennzeichnet, dass** die erste Kammer (7) mit einer Injektionsstelle (25) versehen ist.

18. Vorrichtung nach Anspruch 17, **dadurch gekennzeichnet, dass** die Injektionsstelle (25) auf dem oberen Rand (26) der ersten Kammer (7) vorgesehen ist.

19. Vorrichtung nach einem der Ansprüche 1 bis 18, **dadurch gekennzeichnet, dass** die Infusionsmittel (3) außerdem eine Tropfenzählkammer (28) umfassen.

20. Vorrichtung nach einem der Ansprüche 1 bis 19, **dadurch gekennzeichnet, dass** der Beutel (2) ein Aufhängmittel (31) umfasst, das zum Halten des besagten Beutels (2) in einer Infusionsposition vorgesehen ist, in der das obere Volumen (9) über dem unteren Volumen (10) bzw. über der zweiten Kammer (8) angeordnet ist.

21. Vorrichtung nach einem der Ansprüche 1 bis 20, **dadurch gekennzeichnet, dass** die zweite Kammer (8) ein Luftvolumen enthält.
